# EUROPÄISCHE PATENTSCHRIFT

(11) **EP 1 077 684 B1**
(45) Veröffentlichungstag und Bekanntmachung des Hinweises auf die Patenterteilung: **06.08.2003**
(21) Anmeldenummer: 99922151.8
(22) Anmeldetag: 03.05.1999
(51) Int. Cl.: A61K 9/46

(54) **BRAUSEZUBEREITUNGEN**
EFFERVESCENT PREPARATIONS
PREPARATIONS EFFERVESCENTES

(30) Priorität: 15.05.1998 DE 19822036
(43) Veröffentlichungstag der Anmeldung: 28.02.2001
(73) Patentinhaber: Bayer Aktiengesellschaft, 51368 Leverkusen (DE)
(72) Erfinder: WALTER, Reinhard, Granger, IN 46530 (US); OHAGE-SPITZLEI, Petra, D-51371 Leverkusen (DE)
(86) Internationale Anmeldenummer: EP9902969
(87) Internationale Veröffentlichungsnummer: WO99059553

(56) Entgegenhaltungen:
- WO-A-92/15197
- GB-A- 2 307 857
- US-A- 3 653 914
- US-A- 4 004 036
- US-A- 5 223 246

## Beschreibung

Die Erfindung betrifft ein Verfahren zur Herstellung Arzneimittel enthaltender Brausezubereitungen unter mindestens teilweisem Schmelzen einer Zubereitungskomponente und nach diesem Verfahren erhältliche Brausezubereitungen.

Brausezubereitungen, wie z.B. Brausepulver oder Brausetabletten, sind beispielsweise bei Wirkstoffen mit langer Resorptionszeit oder mit einer Tendenz zu Magenschleimhautreizungen eine Formulierungsform, die die genannten nachteiligen Wirkstoffeigenschaften mildern kann. Arzneimittel enthaltende Brausezubereitungen erfreuen sich deshalb steigender Beliebtheit. Sie werden üblicherweise in 3 bis 4 Stufen hergestellt, nämlich durch
a) Granulation des Brausesatzes aus CO₂-Spender und CO₂-freisetzender saurer Komponente,
b) Mischen der übrigen Komponenten (Wirkstoffe und weitere Hilfsstoffe),
c) Vereinigung der aus den Verfahrensschritten a) und b) erhaltenen Komponenten und gegebenenfalls
d) Tablettieren der in Schritt c) erhaltenen Mischung.

Da sowohl der CO₂-Spender als auch die saure Komponente für eine Direkttablettierung schlecht geeignet sind, hat man in der Vergangenheit die Komponenten des Brausesatzes, gegebenenfalls in Kombination mit dem Wirkstoff, vor der Tablettierung einem Granulationsverfahren unterworfen; vgl. z.B. DE-OS 22 16 072. Die Stabilität der auf diese Weise hergestellten Brausetabletten ist aber nach wie vor nicht zufriedenstellend. Besonders die Mitverwendung von Puffersubstanzen und Aromen (die ja meist aus vielen verschiedenen Einzelverbindungen bestehen) ergibt eine Wasserempfindlichkeit, die beim Lagern zu Verfärbungen, Aufblähungen und Abbaureaktionen führt. Zur Vermeidung dieser unerwünschten Reaktionen werden Brausezubereitungen oft in Metallfolien eingeschweißt. Wenn diese Maßnahme die Lagerstabilität auch verlängert, kann ein Aufblähen der Metallfolienbeutel bei längerem Lagern nicht sicher verhindert werden.

Überraschenderweise wurde nun gefunden, daß die Stabilität Arzneimittel enthaltender Brausezubereitungen durch ein Verfahren gesteigert werden kann, bei dem eine Zubereitungskomponente geschmolzen wird.

Gegenstand der Erfindung ist also ein Verfahren zur Herstellung Arzneimittel enthaltender Brausezubereitungen aus
A. Brausesatz enthaltend
   (i) CO₂-Spender und
   (ii) saure Komponente,
B. pharmazeutischem Wirkstoff und
C. Hilfsstoff,
dadurch gekennzeichnet, daß man
mindestens eine der beiden Komponenten A(i), A(ii) und gegebenenfalls weitere Brausezubereitungskomponenten in geschmolzenem C) Zucker und/oder Zuckeralkohol und/oder Zucker-Ersatzstoff dispergiert und die resultierende Mischung gegebenenfalls tablettiert.

Die Erfindung beinhaltet, daß man gegebenenfalls eine, mehrere oder alle restlichen Brausezubereitungskomponenten in der Schmelze dispergiert.

Ein bevorzugtes Verfahren ist dadurch gekennzeichnet, daß man
- eine Schmelze aus Komponente A (i) und/oder A (ii) und C) schmelzbarem Zucker und/oder Zuckeralkohol und/oder Zucker-Ersatzstoff während oder nach dem Abkühlen zerkleinert,
- das zerkleinerte Produkt mit Wirkstoff B, mit der gegebenenfalls noch fehlenden Komponente (i) oder (ii) des Brausesatzes A und gegebenenfalls weiteren Hilfsstoffen C mischt und gegebenenfalls
- die resultierende Mischung tablettiert.

Bevorzugte CO₂-Spender A (i) umfassen Alkali- und Erdalkalicarbonate und -hydrogencarbonate, insbesondere Natrium- und Kaliumcarbonat und -hydrogencarbonat sowie Magnesium- und Calciumcarbonat.

Als saure Komponente A (ii), die der Freisetzung von Kohlendioxid aus dem CO₂-Spender A (i) dient, eignen sich alle physiologisch unbedenklichen Säuren (sog. "Genußsäuren"), die stark genug sind, Kohlendioxid aus der Komponente A (i) freizusetzen; solche Säuren besitzen einen ersten Gleichgewichtsexponenten pKs von 1 bis 7, vorzugsweise 2 bis 6 (bei 25°C). Bevorzugte saure Komponenten A (i) umfassen Ascorbinsäure und mehrbasische Carbonsäuren mit 3 bis 8, vorzugsweise 4 bis 6, C-Atomen und 2 bis 4 Carboxylgruppen pro Molekül, wie z.B. Vitamin C, Äpfelsäure, Citronensäure, Weinsäure und deren Mischungen.

### Geeignete pharmazeutische Wirkstoffe C umfassen

Analgetika wie Ibuprofen, Ketoprofen, Paracetamol, Acetylsalicylsäure, COX₂-Hemmer wie Nimesulid, Meloxicam, Naproxen, Propyphenazon, Metamizol, Antacida wie Hydrotalcit, Magaldrat, Calciumcarbonat,
Antiasthmatika/Broncholytika wie Salbutamol, Tulobuterol, Terbutalin, Cromoglicinsäure, Ketotifen, Theophyllin,
Antibiotika wie Chinolone, Tetracycline, Cephalosporine, Penicilline, Makrolide, Sulfonamide, Polypeptide,
Psychopharmaka wie Benzodiazepine, Haloperidol, Amitriptylin, Carbamazepin, Antirheumatika wie Phenylbutazon, Indometacin, Diclofenac, Piroxicam, Antidiabetika wie Metformin, Glibenclamid, Acarbose, Glisoxepid, Antiallergika/Antihistaminika wie Astemizol, Terfenadin, Loratadin, Clemastin, Bamipin, Cetirizin,
Antihypotonika wie Etilefrin, Norfenefrin, Dihydroergotaminmesilat,
Antitussiva wie Codein, Dextromethorphan, Clobutinol, Dropropizin,
Antihypertonika wie Betablocker wie Propranolol, Atenolol, Metoprolol, Prazosin,
Antihypertonika wie Calciumantagonisten wie Nifedipin, Nitrendipin, Diltiazem, Verapamil, Felodipin, Nimodipin,
Laxantia wie Natriumpicosulfat, Lactulose, Lactitol,
Mucolytika/Expectorantia wie Ambroxol, Bromhexin, Guaifenesin, Acetylcystein, Carbocistein,
H2-Blocker wie Ranitidin, Famotidin, Pirenzepin,
Lokalanaesthetika wie Benzocain, Lidocain, Procain,
Antiemetika/Prokinetika wie Metoclopramid, Domperidon, Meclozin, Dimenhydrinat,
Lipidsenker wie Fenofibrat, Bezafibrat, Pravastatin, Fluvastatin,
gegen Migräne wirksame Mittel wie Coffein, Dihydroergotamin, Ergotamin, Sumatriptan, Pizotifen,
Sympathomimetika wie Pseudoephedrin, Pholedrin,
Vitamine und Mineralien.

Die Hilfsstoffe C, die im erfindungsgemäßen Verfahren wenigstens teilweise schmelzen sollen, besitzen als Einzelsubstanz und/oder in Mischungen vorzugsweise Schmelzpunkte von 30 bis 200, vorzugsweise von 40 bis 160°C. Bevorzugte solche Hilfsstoffe sind wasserlöslich, d.h. sie besitzen bei 20°C im allgemeinen eine Wasserlöslichkeit von mindestens 10, vorzugsweise mindestens 30 und insbesondere mindestens 40 g/100 ml Wasser.

Schmelzbare Zucker C umfassen z.B. Monosaccharide wie Glucose, Mannose, Galactose, Arabinose, Xylose, Ribose und Disaccharide wie Saccharose, Lactose, Maltose. Für die Erfindung bevorzugte Zuckeralkohole C umfassen Xylit, Mannit, Sorbit, Isomalt, Lactitol, Erythrit, Threit, Ribit, Arabit und Dulcit. Bevorzugte solche Zuckeralkohole werden beispielsweise in der EP-PS 435 450 beschrieben. Der Begriff "Zucker-Ersatzstoffe" im Sinne der Erfindung schließt Zuckeralkohole nicht ein. Bevorzugte Zucker-Ersatzstoffe C umfassen Acesulfam, Aspartam, Saccharin, Natriumcyclamat.

Weitere Hilfsstoffe C umfassen Aromen, Süßstoffe, Schmiermittel, Fließreguliermittel, Sprengmittel und Füllstoffe, wie z.B. Stärke und Stärkederivate, Cellulose und Cellulosederivate, Polyethylene.

Die erfindungsgemäß erhältlichen Brausezubereitungen können die Komponenten in den unterschiedlichsten Mengenverhältnissen enthalten; bevorzugte Brausezubereitungen enthalten (jeweils in Gewichtsteilen)
A: 5 bis 95, vorzugsweise 10 bis 80,
B: 5 bis 95, vorzugsweise 40 bis 60,
C: 1 bis 60, vorzugsweise 15 bis 30 (Zucker, Zuckeralkohol bzw. Zucker-Ersatzstoff) und gegebenenfalls
   1 bis 50, vorzugsweise 5 bis 15 (andere Hilfsstoffe).

Der Brausesatz A enthält vorzugsweise
30 bis 70 Gew.-% CO₂-Spender und
70 bis 30 Gew.-% saure Komponente,
jeweils bezogen auf A.

Die Schmelze aus Brausesatz-A(-Komponente) und schmelzbarem Zucker und/oder Zuckeralkohol und/oder Zucker-Ersatzstoff C kann beispielsweise durch Zugabe von Brausesatz-A (-Komponenten) in eine Schmelze von Zucker und/oder Zuckeralkohol und/oder Zucker-Ersatzstoff C oder durch Aufschmelzen einer Mischung aus Brausesatz-A (-Komponenten) und Zucker und/oder Zuckeralkohol und/oder Zucker-Ersatzstoff C hergestellt werden.

Das erfindungsgemäße Verfahren kann aber auch so ausgeführt werden, daß man sämtliche Komponenten der Brausezubereitung mit dem geschmolzenen Zucker und/oder Zuckeralkohol und/oder Zucker-Ersatzstoff zwecks Dispergieren in Kontakt bringt, sei es durch Vormischen aller Komponenten und gemeinsames Erhitzen, sei es durch Schmelzen von Zucker und/oder Zuckeralkohol und/oder Zucker-Ersatzstoff und Dispergieren der restlichen Komponenten (gleichzeitig oder nacheinander) in der Schmelze. Selbstverständlich können auch Mischformen der beschriebenen Verfahrensvarianten verwendet werden.

Die Schmelze kann auf nahezu beliebige Weise hergestellt werden:
So kann man ohne weiteres in heizbaren Rührgefäßen arbeiten. Man kann auch nach einem Schmelzgranulierungsverfahren arbeiten, wie es z.B. in der WO 92/6679 beschrieben ist. Ein bevorzugtes Verfahren ist die Schmelzextrusion, wie sie z.B. in der EP-A 686 392 beschrieben ist Für die Extrusion können handelsübliche Ein- und Zweiwellenextruder eingesetzt werden. Dabei können die Einsatzstoffe der Extrusion über eine Dosierwaage zugeführt werden. Die Massetemperatur kann 30 bis 200°C betragen. In Abhängigkeit von Düsenöffnung (vorzugsweise 0,5 bis 5 mm) und Drehzahl (vorzugsweise 5 bis 400 Umdrehungen/Minute) kann der Druck vorzugsweise 2 bis 200 bar betragen. Der Durchsatz kann innerhalb weiter Grenzen schwanken, beträgt aber vorzugsweise 1 bis 100 kg/Stunde. Die extrudierten Stränge werden gegebenenfalls gekühlt. Nach dem Zerkleinern können sie mit Wirkstoff B und gegebenenfalls weiteren Hilfsstoffen C gemischt und gegebenenfalls tablettiert werden.

Beim erfindungsgemäßen Verfahren wird vorzugsweise weder Wasser noch ein unter Verarbeitungsbedingungen flüchtiges organisches Lösungsmittel eingesetzt, d.h. vorzugsweise arbeitet man wasser- und lösungsmittelfrei. Mit anderen Worten: Man arbeitet gerade nicht so, wie es in der DE-OS 22 16 072 oder in
Acta Pharm. Suec., 24, (2), 84, 1987
Drug Dev. Ind. Pharm. 13, (9-11), 1891-1913, 1987
Drug Dev. Ind. Pharm. 14, (13), 1791-98, 1988
beschrieben ist.

Das erfindungsgemäße Verfahren kann kontinuierlich oder diskontinuierlich betrieben werden.

Durch das erfindungsgemäße Verfahren werden die Komponente A (i) und/oder A (ii) und gegebenenfalls weitere Brausezubereitungskomponenten in dem schmelzbaren Zucker, Zuckeralkohol bzw. Zucker-Ersatzstoff C dispergiert, d.h. die schmelzbare Komponente C bildet eine Matrix, in der A (i) und/oder A (ii) und gegebenenfalls weitere Brausezubereitungskomponenten eingebettet sind.

Weiterer Gegenstand der Erfindung sind also Brausezubereitungen aus
A. Brausesatz enthaltend
   (i) CO₂-Spender und
   (ii) saure Komponente,
B. pharmazeutischem Wirkstoff und
C. Hilfsstoff,
dadurch gekennzeichnet, daß Hilfsstoff C schmelzbaren Zucker und/oder Zuckeralkohol und/oder Zucker-Ersatzstoff enthält und die Komponente A (i) und/oder A (ii) in einer Matrix aus geschmolzenem Zucker und/oder Zuckeralkohol und/oder Zucker-Ersatzstoff dispergiert ist.

Die Prozentangaben der nachfolgenden Beispiele beziehen sich jeweils auf das Gewicht.

### Beispiele

### Beispiel 1

Brausezubereitung aus getrennt extrudierten Komponenten A (i) und A (ii)

### Extrudat I

Mannitol und Natriumbicarbonat werden in den in der Tabelle angezeigten Verhältnissen gemischt. Die Mischung wird auf einem Doppelschneckenextruder (Leistriz Micro 27/40D.) bei einer Drehzahl von 30 upm und einem Düsendurchmesser vom 1 mm verarbeitet. Die Düsen sind um den äußeren Durchmesser der Schnecken angeordnet. Mischzonen und Düsentemperatur betragen 80°C. Das Extrudat wird auf einem Kühlband gekühlt und anschließend mit einem Oscillationssieb zerkleinert.

### Extrudat II

Mannitol, Citronensäure, Natriumcitrat werden gemischt und extrudiert und wie oben weiterverarbeitet.

| | Extrudat I: | Extrudat II: |
|---|---|---|
| Mannitol | 60 % | 60 % |
| Natriumbicarbonat | 40 % | |
| Citronensäure | | 6,7 % |
| Natriumcitrat | | 33,3 %. |

Bezogen auf eine Einzeldosierung werden von Extrudat I 125 mg, von Extrudat II 150 mg mit 500 mg Acetylsalicylsäure, 5 mg Aspartam und 30 mg Orangenaroma gemischt und in ein Sachet abgefüllt.

### Beispiel 2

Analog Beispiel 1 werden Extrudat I und Extrudat II bei einer Temperatur von 70°C, einem Düsendurchmesser von 0,8 mm und einer Drehzahl von 26 upm extrudiert.

| | Extrudat I: | Extrudat II: |
|---|---|---|
| Xylitol | 60 % | 60 % |
| Natriumbicarbonat | 40 % | |
| Citronensäure | | 40 % |

Bezogen auf eine Einzeldosierung werden von Extrudat I 125 mg, von Extrudat II 150 mg mit 500 mg Acetylsalicylsäure, 4 mg Saccharin und 30 mg Mandarinenaroma gemischt und in ein Sachet abgefüllt.

### Beispiel 3

Analog Beispiel 2 werden Extrudat I und Extrudat II bei einer Temperatur von 60°C, einem Düsendurchmesser von 1 mm und einer Drehzahl von 35 upm extrudiert

| | Extrudat I: | Extrudat II: |
|---|---|---|
| Xylitol | 30 % | 30 % |
| Natriumbicarbonat | 70 % | |
| Citronensäure | | 70 % |

Bezogen auf eine Einzeldosierung werden jeweils 125 mg von Extrudat I und II mit 150 mg Ascorbinsäure und 2,5 mg Chlorpheniraminhydrogenmaleat gemischt und in ein Sachet eingefüllt.

### Beispiel 4

Analog Beispiel 2 werden Extrudat I und Extrudat II bei einer Temperatur von 60°C, einem Düsendurchmesser von 2 mm und einer Drehzahl von 35 upm extrudiert

| | Extrudat I: | Extrudat II: |
|---|---|---|
| Isomalt | 60 % | |
| Xylitol | | 60 % |
| Kaliumbicarbonat | 40 % | |
| Ascorbinsäure | | 40 % |

Bezogen auf eine Einzeldosierung werden von Extrudat I 125 mg, von Extrudat II 250 mg mit 500 mg Acetylsalicylsäure, 5 mg Saccharin, 2 mg Aspartam und 30 mg Orangenaroma gemischt und in ein Sachet abgefüllt.

### Beispiel 5

Analog Beispiel 1 werden Extrudat I und Extrudat II bei einer Temperatur von 60°C, einem Düsendurchmesser von 1 mm und einer Drehzahl von 35 upm extrudiert.

| | Extrudat I: | Extrudat II: |
|---|---|---|
| Mannitol | 60 % | 60 % |
| Natriumbicarbonat | 20 % | |
| Calciumcarbonat | 20 % | |
| Ascorbinsäure | | 40 % |

Bezogen auf eine Einzeldosierung werden jeweils von Extrudat I 1500 mg und Extrudat II 750 mg mit 5 mg Aspartam und 10 mg Johannisbeeraroma gemischt und in ein Sachet abgefüllt.

### Beispiel 6

Formulierung mit nur einer extrudierten Komponente, nämlich A (ii)

| | |
|---|---|
| Extrudat II aus Beispiel 2 | 1200 mg |
| Famotidin | 10 mg |
| Natriumbicarbonat | 400 mg |
| Natriumcarbonat | 100 mg |
| Magnesiumstearat | 20 mg |

Analog Beispiel 1 wird nur die Säure-Komponente extrudiert und die alkalische Brausekomponente und der Wirkstoff werden dazugemischt. Anschließend wird Magnesiumstearat nachgemischt. Diese Mischung wird zu einer Brausetablette verpresst.

### Beispiel 7

Gemeinsame Extrusion von A (i) und A (ii)

| | |
|---|---|
| Xylitol | 60 % |
| Na-citrat | 14 % |
| Natriumhydrogencarbonat | 23 % |
| Zitronensäure | 3 % |

Herstellverfahren:
A) Extrusion analog Beispiel 1, oder
B) Xylitol aufschmelzen auf ca. 120°C und die Komponenten nacheinander zudosieren und einrühren. Nach Abkühlung wird der Schmelzkuchen zerkleinert.

Bezogen auf eine Einzeldosierung werden jeweils 600 mg des erhaltenen Extrudats, 200 mg Acetylcystein und 10 mg Zitronenaroma gemischt. Die erhaltene Pulvermischung wird in ein Sachet abgefüllt.

### Beispiel 8

Eine Mischung aus 54 % Xylitol, 6 % Pseudoephedrin, 14 % Natriumcitrat, 23 % Natriumhydrogencarbonat und 3 % Zitronensäure werden analog Beispiel 1 extrudiert. Das Extrudat wird zerkleinert und abgefüllt.

### Stabilitätsvergleich von ASS-haltigen Brauseformulierungen

Bestimmung des Abbauproduktes Salicylsäure (SAS) nach 3 Monaten bei 25°C Lagerung in wasserdampfdichter Verpackung

| | Anfangsgehalt SAS | SAS-Gehalt nach 3 Monaten |
|---|---|---|
| ASS-Brausegranulat, aromatisiert | 0,02 % | 1,61 % |
| ASS-Brausegranulat (extrudiert), aromatisiert^{xx} | 0,04 % | 0,18 % |
| ASS-Brausetablette, aromatisiert* | 0,3 % | 1,83 % |
| ASS-Brausetablette, unaromatisiert* | 0,17 % | 0,8 % |

| | | |
|---|---|---|
| * Granulat ist mit herkömmlicher Technologie hergestellt (Vergleichsversuch) | | |
| ^{xx} erfindungsgemäß | | |

## Patentansprüche

1. Verfahren zur Herstellung Arzneimittel enthaltender Brausezubereitungen aus
A. Brausesatz enthaltend
(i) CO₂-Spender und
(ii) saure Komponente,
B. pharmazeutischem Wirkstoff und
C. Hilfsstoff,
**dadurch gekennzeichnet, daß** man
mindestens eine der beiden Komponenten A(i), A(ii) und gegebenenfalls weitere Brausezubereitungskomponenten in geschmolzenem C) Zucker und/oder Zuckeralkohol und/oder Zucker-Ersatzstoff dispergiert und die resultierende Mischung gegebenenfalls tablettiert.

2. Verfahren nach Anspruch 1, wonach man
- eine Schmelze aus Komponente A (i) und/oder A (ii) und C schmelzbarem Zucker, Zuckeralkohol und/oder Zucker-Ersatzstoff während oder nach dem Abkühlen zerkleinert,
- das zerkleinerte Produkt mit Wirkstoff B, mit der gegebenenfalls noch fehlenden Komponente (i) oder (ii) des Brausesatzes A und gegebenenfalls weiteren Hilfsstoffen C mischt und gegebenenfalls
- die resultierende Mischung tablettiert.

3. Verfahren nach Anspruch 1, wonach zum Schmelzen ein Extruder verwendet wird.

4. Verfahren nach Anspruch 1, wonach der pharmazeutische Wirkstoff B aus der Gruppe Analgetika, Antacida, Antiasthmatika/Broncholytika, Antibiotika, Psychopharmaka, Antidiabetika, Antiallergika/Antihistaminika, Antihypotonika, Antitussiva, Laxantia, Mucolytika/Expectorantia, H2-Blocker, Lokalanaesthetika, Antiemetika/Prokinetika, Lipidsenker, gegen Migräne wirksame Mittel, Sympathomimetika, Vitamine, Mineralien ausgewählt ist.

5. Verfahren nach Anspruch 1, wonach die Temperatur der Schmelze 30 bis 200°C beträgt.

6. Verfahren nach Anspruch 1, wonach die Temperatur der Schmelze 40 bis 160°C beträgt.

7. Brausezubereitung aus
A. Brausesatz enthaltend
(i) CO₂-Spender und
(ii) saure Komponente,
B. pharmazeutischem Wirkstoff und
C. Hilfsstoff,
**dadurch gekennzeichnet, daß** Hilfsstoff C schmelzbaren Zucker und/oder Zuckeralkohol und/oder Zucker-Ersatzstoff enthält und die Komponente A (i) und/oder A (ii) in einer Matrix aus geschmolzenem Zucker und/oder Zuckeralkohol und/oder Zucker-Ersatzstoff dispergiert ist.

## Claims

1. Process for producing medicament-containing effervescent preparations consisting of
A. effervescent composition containing
(i) CO₂ donor and
(ii) acidic component,
B. pharmaceutical active substance and
C. ancillary substance,
**characterized in that**
at least one of the two components A(i) and A(ii) and, where appropriate, other effervescent preparation components are dispersed in molten C) sugar and/or sugar alcohol and/or sugar substitute, and the resulting mixture is tabletted where appropriate.

2. Process according to Claim 1, wherein
- a melt consisting of component A(i) and/or A(ii) and C fusible sugar, sugar alcohol and/or sugar substitute is comminuted during or after the cooling,
- the comminuted product is mixed with active substance B, with component (i) or (ii), which is still missing where appropriate, of the effervescent composition A and, where appropriate, with further ancillary substances C and, where appropriate,
- the resulting mixture is tabletted.

3. Process according to Claim 1, wherein an extruder is used for the melting.

4. Process according to Claim 1, wherein the pharmaceutical active substance B is selected from the group of analgesics, antacids, antiasthmatics/bronchospasmolytics, antibiotics, psychopharmaceuticals, antidiabetics, antiallergics/antihistamines, antihypotensives, antitussives, laxatives, mucolytics/expectorants, H2 blockers, local anaesthetics, antiemetics/prokinetics, lipid lowering agents, agents effective for migraine, sympathomimetics, vitamins, minerals.

5. Process according to Claim 1, wherein the temperature of the melt is 30 to 200°C.

6. Process according to Claim 1, wherein the temperature of the melt is 40 to 160°C.

7. Effervescent preparation consisting of
A. effervescent composition containing
(i) CO₂ donor and
(ii) acidic component,
B. pharmaceutical active substance and
C. ancillary substance,
**characterized in that** ancillary substance C contains fusible sugar and/or sugar alcohol and/or sugar substitute, and component A(i) and/or A(ii) is dispersed in a matrix of molten sugar and/or sugar alcohol and/or sugar substitute.

## Revendications

1. Procédé de préparation de préparations effervescentes contenant un médicament, constituées :
A. d'une composition effervescente contenant
(i) un distributeur de CO₂, et
(ii) un composant acide,
B. d'une substance active pharmaceutique, et
C. d'un adjuvant,
**caractérisé en ce qu'**on disperse
au moins un des deux composants A(i), A(ii) et le cas échéant d'autres composants de préparation effervescente dans du sucre et/ou alcool de sucre et/ou ersatz de sucre C) fondu et on met le cas échéant en comprimés le mélange résultant.

2. Procédé selon la revendication 1, d'après lequel :
- on broie pendant ou après le refroidissement une fusion du composant A(i) et/ou A(ii) et de sucre, alcool de sucre et/ou ersatz de sucre C pouvant fondre;
- on mélange le produit broyé avec la substance active B, avec laquelle on mélange le cas échéant encore des composants manquants (i) ou (ii) de la composition effervescente A et le cas échéant d'autres adjuvants C et, le cas échéant,
- on met en comprimés le mélange résultant.

3. Procédé selon la revendication 1, dans lequel on utilise pour la fusion une extrudeuse.

4. Procédé selon la revendication 1, dans lequel la substance active pharmaceutique B est sélectionnée dans le groupe des analgésiques, antiacides, antiasthmatiques/broncholitiques, antibiotiques, agents psychopharmacologiques, antidiabétiques, antiallergiques/antihistaminiques, antihyptoniques, antifussifs, laxatifs, mucolytiques/expectorants, agents de blocage H2, anesthésiques locaux, antiémétiques/prokinétiques, réducteurs de lipide, substances actives contre les migraines, les sympathomimétiques, vitamines, minéraux.

5. Procédé selon la revendication 1, dans lequel la température de la fusion est de 30 à 200°C.

6. Procédé selon la revendication 1, dans lequel la température de la fusion est de 40 à 160°C.

7. Préparation effervescente constituée :
A. d'une composition effervescente contenant
(i) un distributeur de CO₂, et
(ii) un composant acide,
B. d'une substance active pharmaceutique, et
C. d'un adjuvant,
**caractérisée en ce que** l'adjuvant C contient du sucre et/ou alcool de sucre et/ou ersatz de sucre pouvant fondre et le composant A(i) et/ou A(ii) est dispersé dans une matrice de sucre et/ou alcool de sucre et/ou ersatz de sucre fondu.
